# EUROPEAN PATENT APPLICATION

(11) **EP 2 345 700 A2**
(43) Date of publication of application: **20.07.2011**
(21) Application number: 09820701.2
(22) Date of filing: 23.09.2009
(51) Int. Cl.: C08L 89/00

(54) **POLYMER SUPPORT BONDED TO AGGLOMERATION NUCLEI**

(30) Priority: 13.10.2008 KR 20080100385
(71) Applicant: SNU R&DB Foundation, Seoul 151-742 (KR)
(72) Inventor: LEE, Yoon-Sik, Anyang-si Gyeonggi-do 431-714 (KR); PAIK, Seung Ryeoul, Bucheon-si Gyeonggi-do 420-730 (KR)
(74) Representative: Long, Giorgio
(86) International application number: PCT/KR2009/005402
(87) International publication number: WO 2010/044551

(57) **Abstract**

The present invention relates to a seed-conjugated polymer support. In particular, the present invention is directed to a seed-conjugated polymer support for aggregating biomolecules, a method for preparing the same, and a method for removing β-2-microglobulin.

## Description

### Technical Field

The present invention relates to a seed-conjugated polymer support. In particular, the present invention is directed to a seed-conjugated polymer support for aggregating biomolecules, a method for preparing the same, and a method for removing β-2-microglobulin.

### Background Art

Amyloid is a fibrous protein aggregate resulted from induction of insoluble supermolecular protein structure formed by cross β-sheet conformation between water-soluble proteins. Amyloid proteins are commonly found in patients undergoing degenerative disease such as Parkinson's disease, dementia, bovine spongiform encephalopathy, etc.

Since metabolic waste products in patients with renal dysfunction are not sufficiently removed from the body, they are to be removed through hemodialysis. However, the metabolic waste products removal efficiency of a hemodialyser is lower than that of a kidney. Especially, β-2-microglobulin which is a part of a major histocompatibility complex class I, is not completely removed by a hemodialyser and, thus, accumulated in the body, thereby being deposited in joint tissues by forming amyloids. This is dialysis-related amyloidosis. Carpal tunnel syndrome, arthritis, cervical arthritis, osteosystoma which may cause fracture, etc. may be induced by the dialysis-related amyloidosis.

It is known that the concentration of β-2-microglobulin in patients undergoing hemodialysis for a long term is about 60 times higher than that of a normal individual. In addition, it has been reported that dialysis-related amyloidosis caused diseases were alleviated when the concentration of β-2-microglobulin in patients decreased to a normal value. Accordingly, much efforts have been made to lower the β-2-microglobulin concentration in serum to be approximately normal by increasing β-2-microglobulin removal efficiency through improving a hemodialyser or by equipping a hemodialyser with an auxiliary chromatographic column having higher ability for adsorbing β-2-microglobulin (Humes HD et al., The future of hemodialysis membranes, Kidney Int., 2006, 69, 1115-1119; Furuyoshi S et al., New adsorbent for extracorporeal removal of β2-microglobulin. In:Amyloid and amyloidosis, New York: Plenum Press, 1988, 629-634; Ameer et al., A novel immunoadsorption device for removing of β2-microglobulin from whole blood, Kidney International, 2001, 59,1544-550).

Commercially available columns such as Luxell™ is filled with polymer (such as agarose or cellulose) based beads and removes β-2-microglobulin by separating proteins by size and by adsorbing proteins by hydrophobicity. However, this has disadvantages in that the amount of removed β-2-microglobulin is not sufficient due to low binding affinity and other proteins having similar sizes are also removed due to non-specificity.

When an antibody is introduced to a polymer support in order to selectively remove β-2-microglobulin, high affinity and specificity toward β-2-microglobulin is obtained. However, it is expensive to produce antibodies and, moreover, antibodies are unstable and easily damaged when they are stored at room temperature for a long time (Shabunina et al., Immunoabsorbent for removal of β2-microglobulin from human blood plasma. Bulletin of experimental biology and medicine, 2001,132, 984-986).

In the case of Luxell™ which is a commercially available column for removing β-2-microglobulin, a polymer support to which hydrophobic carbon chains are introduced is used for enhancing affinity. However, it is disadvantageous that other valuable proteins are removed together with β-2-microglobulin.

Therefore, there is a continuing need, in the art, for a polymer support that can remove only β-2-microglobulin from blood, without removing other useful proteins, by selective binding to β-2-microglobulin.

The present inventors have found that the formation of amyloid is much accelerated when amyloid protein monomer exists together with a fibrous structure made from proteins of the same kind, and have completed the present invention of an amyloid seed-conjugated polymer support which can selectively remove proteins to be removed by conjugating to a polymer support a seed prepared from amyloid made from protein to be removed.

### Technical Problem

The primary object of the present invention is to provide a seed-conjugated polymer support for aggregating biomolecules.

Another object of the present invention is to provide a method for preparing a seed-conjugated polymer support for aggregating biomolecules, comprising: i) reacting a polymer resin to which carboxyl group is introduced with one selected from the group consisting of N-hydroxysuccinimide, glutaraldehyde and epoxy to produce an activated polymer support; and ii) reacting said activated polymer support with a seed for aggregating biomolecules to introduce said seed onto a surface of said polymer support.

Further object of the present invention is to provide a method for removing β-2-microglobulin, comprising: contacting said β-2-microglobulin amyloid seed-conjugated polymer support with a fluid comprising β-2-microglobulin.

### Technical Solution

The aforementioned primary object of the present invention can be achieved by providing a seed-conjugated polymer support for aggregating biomolecules.

According to the present invention, biomolecules may be separated or removed via the seed-conjugated polymer support prepared by conjugating to a polymer support a seed which can aggregate the biomolecules. Preferably, a size of the seed is 1 nm to 10 µm.

Particularly, the seed may be an amyloid seed. The term "an amyloid seed" as used herein refers to a fragment of an amyloid that has a critical size or structure to form a larger amyloid due to its scaffold suitable to form an amyloid and, thus, its ability to grow fast.

In particular, the amyloid seed-conjugated polymer support allows for removing efficiently proteins to be removed, via protein aggregation effect. The protein aggregation effect refers to a phenomenon that the formation of amyloid is much promoted when an amyloid protein monomer exists together with a fibrous structure made from proteins of the same kind. The rate of amyloid formation when a seed is present is much faster than that when a seed is not present. In addition, the amounts of formed amyloids are significantly different, depending upon whether or not a seed is present. The amyloid seed may be any protein which can form an amyloid, including, but not limited to, a β-2-microglobulin amyloid seed.

The polymer support is selected from polystyrene resin, PEG-g-PS resin, TentaGel™ resin, PEGA™ resin, CLEAR™ resin, epoxy resin, phenol resin, phenoxy resin, melamine resin, polyester resin, cellulose resin, agarose resin, chitosan resin or PMMA resin. Preferably, the polymer support may be polystyrene.

Another object of the present invention can be achieved by providing a method for preparing a seed-conjugated polymer support for aggregating biomolecules, comprising: i) reacting a polymer resin to which carboxyl group is introduced with one selected from the group consisting of N-hydroxysuccinimide, glutaraldehyde and epoxy to produce an activated polymer support; and ii) reacting said activated polymer support with a seed for aggregating biomolecules to introduce said seed onto a surface of said polymer support.

The polymer support of the method of the present invention may be selected from polystyrene resin, PEG-g-PS resin, TentaGel™ resin, PEGA™ resin, CLEAR™ resin, epoxy resin, phenol resin, phenoxy resin, melamine resin, polyester resin, cellulose resin, agarose resin, chitosan resin or PMMA resin. Preferably, the polymer support may be polystyrene.

The polymer resin to which carboxyl group is introduced may be prepared according to the conventional method, as described in Example 2.

Preferably, a solvent being used at the step i) of the method of the present invention may be dichloromethane or dimethylformaldehyde. In addition, a catalyst being used at said step i) may be preferably 4-dimethyl-aminopyridine.

Preferably, a reaction temperature at said step i) is 0 - 50°C and a reaction time at said step i) is 2 - 48 hours.

The seed of said step ii) may be an amyloid seed, and the amyloid seed of said step ii) may be a β-2-microglobulin amyloid seed. In addition, a size of said seed is preferably 1 nm to 10 µm.

Further object of the present invention can be achieved by providing a method for removing β-2-microglobulin, comprising: contacting said β-2-microglobulin amyloid seed-conjugated polymer support with a fluid comprising β-2-microglobulin.

It is known in the art that β-2-microglobulin forms, *in vitro,* amyloids only in an acidic condition, while it forms easily amyloids *in vivo.* It is also known that various agents which facilitate fibrillation should be added in order to form amyloids *in vitro* in a neutral condition. The protein aggregation effect is known to promote the formation of amyloids. The efficiency of β-2-microglobulin removal may be increased by using an adsorption column which is filled with the β-2-microglobulin amyloid seed-conjugated polymer support and promotes the formation of β-2-microglobulin amyloids.

That is, in vivo β-2-microglobulin may be removed by inducing the formation of amyloids by conjugating β-2-microglobulin with β-2-microglobulin amyloid seeds. During hemodialysis, β-2-microglobulin may be effectively removed by using an auxiliary column filled with the polymer support of the present invention together with a hemodialyser. Preferably, a size of the β-2-microglobulin amyloid seed may be 1 nm to 10 µm.

The polymer support being used in the method for removing β-2-microglobulin, according to the present invention, may be selected from polystyrene resin, PEG-g-PS resin, TentaGel™ resin, PEGA™ resin, CLEAR™ resin, epoxy resin, phenol resin, phenoxy resin, melamine resin, polyester resin, cellulose resin, agarose resin, chitosan resin, PMMA resin or silica bead. Preferably, the polymer support may be polystyrene.

In order to prevent the immobilized antibody from being adsorbed by other proteins, the polymer support is treated with a blocking solution such as bovine serum albumin (including bovine calf serum or fetal bovine serum, etc.), horse serum, human serum or skim milk. In addition, materials grafted with PEG (polyethylene glycol) or chitosan allow for avoiding the adsorption of other proteins.

When contacting said β-2-microglobulin amyloid seed-conjugated polymer support with a fluid comprising β-2-microglobulin, phosphate buffer solution may be additionally added to the fluid in order to avoid rapid change of pH. At this, it is preferable that the pH is maintained at 6.0 - 8.0. With this, the environment similar to the human body can be maintained.

### Advantageous Effects

The seed-conjugated polymer support makes it possible to remove protein which is to be removed.

In particular, it is possible that abnormal *in vivo* accumulation of β-2-microglobulin is avoided by selective removal of β-2-microglobulin from blood during hemodialysis treatment to patients undergoing renal failure. With this, dialysis-related amyloidosis may be prevented or treated.

### Brief Description of Drawings

Fig. 1 is a graph showing the formation of β-2-microglobulin amyloid by using thioflavin-T.
Fig. 2 shows TEM images of the amyloid seed of the present invention, prepared by sonication.
Fig. 3 shows one embodiment illustrating the conjugation of β-2-microglobulin seed to Hicore bead™, according to the present invention.
Fig. 4 shows SEM images illustrating the conjugation of β-2-microglobulin with the bead onto which the protein seed of the present invention is immobilized.
Fig. 5 confocal microscopic images illustrating the conjugation of β-2-microglobulin with the bead onto which the protein seed of the present invention is immobilized, by detecting fluorescence signals (A: bare Hicore beads; B: bare Hicore beads reacted with β-2-microglobulin; C: seed-conjugated Hicore beads reacted with β-2-microglobulin for 1 day; D: seed-conjugated Hicore beads reacted with β-2-microglobulin for 3 days; and E: seed-conjugated Hicore beads reacted with α-synuclein for 3 days).
Fig. 6 shows the removal of β-2-microglobulin via protein seed-conjugated polymer beads with the lapse of time.

### Best Mode for Carrying Out the Invention

Hereinafter, the present invention will be described in greater detail with reference to the following examples and drawings. The examples and drawings are given only for illustration of the present invention and not to be limiting the present invention.

### Example 1. Preparation of β-2-microglobulin amyloid seed to be used as a seed

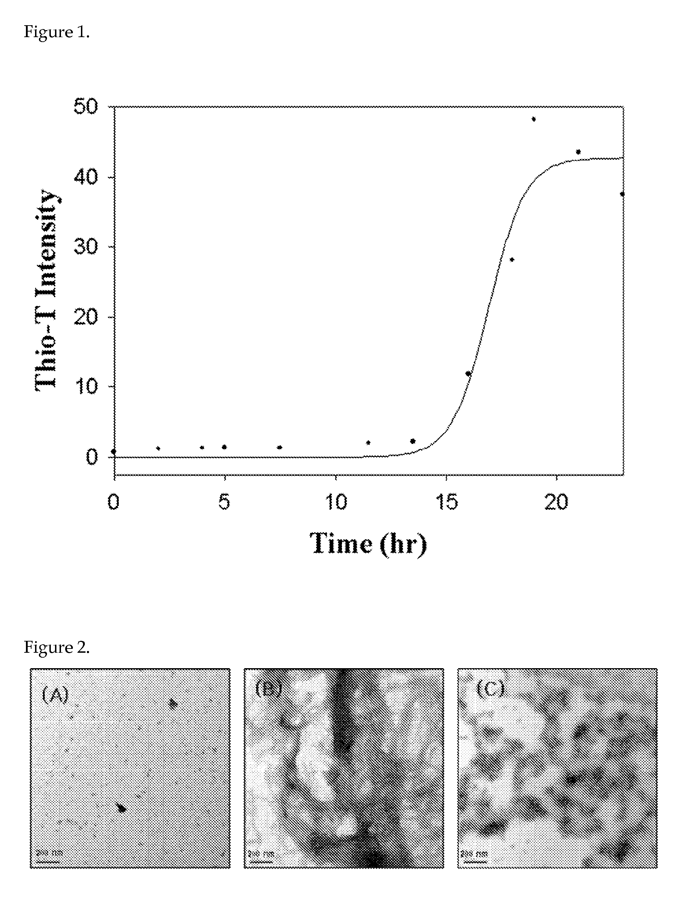

1 mg/ml of purified β-2-microglobulin monomers were added to 20 mM of chloride phosphate buffer solution containing 0.15 M of sodium chloride at pH 2.5 and, then, amyloid formation reaction was proceeded at 37°C for more than 2 weeks. The formation of amyloid fibrils was monitored by using thioflavin-T which is a fluorescent dye and widely used for measuring the degree of amyloid formation from proteins (Fig. 1). After sufficiently mature amyloids were confirmed, amyloid seeds with a size of about 10 nm were obtained by fragmentation of amyloids using sonication and fitration of the amyloid fragments (Fig. 2).

### Example 2. Preparation of a polymer support

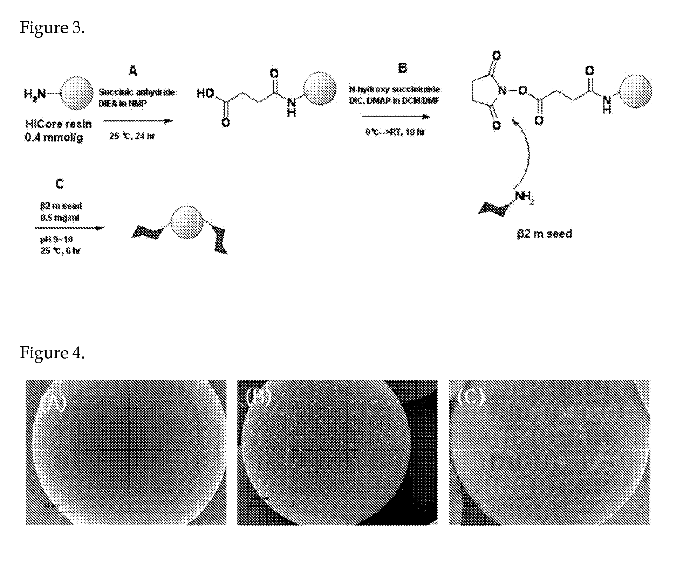

Hicore resins (BeadTech, Inc., Seoul, Korea) which are used for supports for solid phase protein synthesis were employed as polymer supports. At first, the amine groups of Hicore beads were reacted with succinic anhydride in N-methyl-2-pyrrolidone at room temperature for 24 hours, thereby introducing carboxyl groups and, then, N-hydroxysuccinimide was introduced by reacting with N,N'-diisopropyl-carbodiimi d e under the catalyst of f 4-dimethyl-aminopyridine. Dichloromethane and dimethylformamide were used as solvents. The reaction temperature was kept at 0°C in an ice bateh and, after 1 hour, the ice bath was removed and the reaction was proceeded at room temperature for 18 hours. After being activated as N-hydroxysuccinimide ester, polymer supports including chemically binded ligands were obtained by condensation reaction with amine groups of β-2-microglobulin seed prepared by sonication (Fig. 3).

### Example 3. Removal of β-2-microglobulin in a solvent by using seed-conjugated polymer support

The seed-conjugated polymer beads of Example 2 were reacted with 1 % BSA (bovine serum albumin) solution (0.4 mmol NH₂/g bead) at room temperature for 1 hour so as to minimize the non-specific protein adsorption at the surface of the polymer support. The thus treated beads were added to 200 ml of 20 mM chloride phosphate buffer solution (pH 7.5) containing 1 mg/ml of β-2-microglobulin and the reaction was proceeded in this condition. During the reaction at 37°C in a stirred incubator, the amount of β-2-microglobulin bound to the seed with the lapse of time was measured. As a result, it was confirmed by quantitative and qualitative analyses that β-2-microglobulin was selectively bound to the seed-conjugated polymer bead, with the lapse of time. It was also confirmed that β-2-microglobulin was well bound to the polymer support, both at pH 6.5 and pH 7.0.

### 1) Qualitative analysis by fluorescence

FITC (fluorescein isothiocyanate)-labeled β-2-microglobulin monomers were reacted with protein seed-conjugated beads in 20 mM phosphate buffer solution (pH 7.4) at 37°C for 3 days and, then, the β-2-microglobulin bound to the bead were observed with a confocal microscope (Fig. 4).

The left image of Fig. 4 is a confocal microscopic image of the bare polymer bead which was prepared by reacting a bare polymer bead with FITC for 72 hours, the middle image of Fig. 4 is a confocal microscopic image of the β-2-microglobulin coupled polymer bead which was prepared by reacting a seed-conjugated bead with FITC for 24 hours, and the right image of Fig. 4 is a confocal microscopic image of the β-2-microglobulin coupled polymer bead which was prepared by reacting a seed-conjugated bead with FITC for 72 hours.

Although the bare beads were reacted for 3 days, only the weak autofluorescence emitted from the beads and the very small amount of the coupled β-2-microglobulin was detected. In contrast, in the case of the seed-conjugated bead, very clear and intense fluorescence was detected after reacting for only 24 hours, which confirms that β-2-microglobulin was selectively coupled to the bead.

### 2) Quantitative analysis

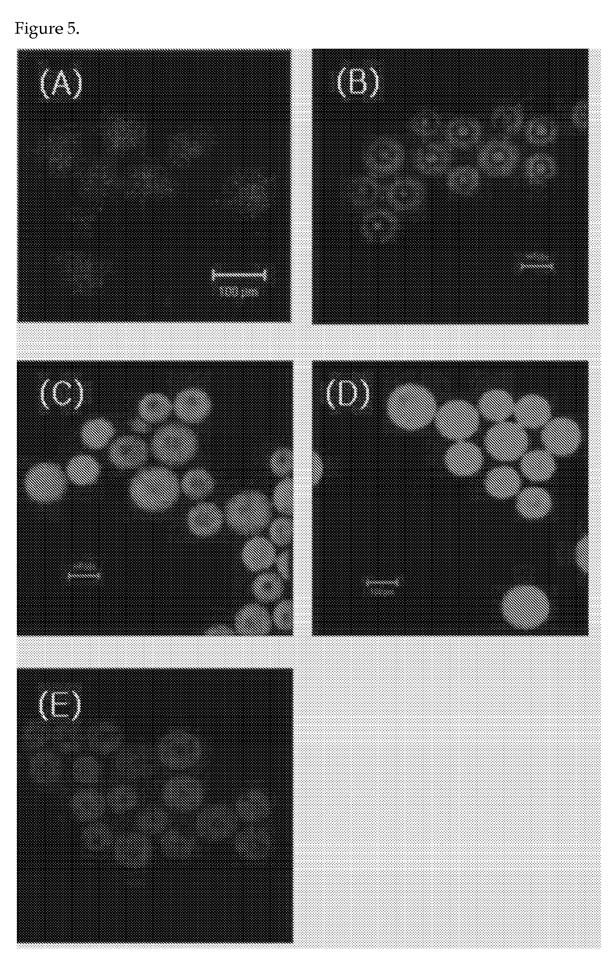

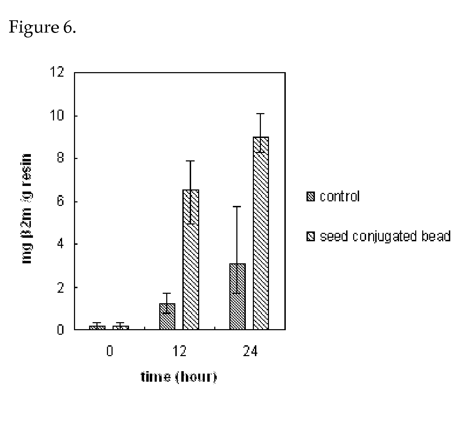

For quantitative analysis, the protein seed-conjugated beads were added to a 20 mM phosphate buffer solution (pH 7.4) containing β-2-microglobulin, and the mixture was stirred at 37°C. Then, the β-2-microglobulin concentrations of the mixture with the lapse of time were analyzed with Bradford assay and the results are shown in Fig. 5. As shown in Fig. 5, the β-2-microglobulin in the mixture was decreased with the lapse of time. From this, it is confirmed that the seed-conjugated bead can remove β-2-microglobulin. In addition, according to the above results, the estimated removal rates of β-2-microglobulin are shown as a graph in Fig. 6. As indicated in Fig. 6, the removal rate of β-2-microglobulin increased with time, which means that the seed effect for amyloid formation is still maintained in the immobilizing support.

## Claims

1. A seed-conjugated polymer support for aggregating biomolecules.

2. The seed-conjugated polymer support of Claim 1, wherein said seed is an amyloid seed.

3. The seed-conjugated polymer support of Claim 2, wherein said amyloid seed is a β-2-microglobulin amyloid seed.

4. The seed-conjugated polymer support of Claim 1, wherein a size of said seed is 1 nm to 10 µm.

5. The seed-conjugated polymer support of Claim 1, wherein said polymer support is selected from the group consisting of polystyrene resin, PEG-g-PS resin, TentaGel™ resin, PEGA™ resin, CLEAR™ resin, epoxy resin, phenol resin, phenoxy resin, melamine resin, polyester resin, cellulose resin, agarose resin, chitosan resin and PMMA resin.

6. A method for preparing a seed-conjugated polymer support for aggregating biomolecules, comprising:
i) reacting a polymer resin to which carboxyl group is introduced with one selected from the group consisting of N-hydroxysuccinimide, glutaraldehyde and epoxy to produce an activated polymer support; and
ii) reacting said activated polymer support with a seed for aggregating biomolecules to introduce said seed onto a surface of said polymer support.

7. The method of Claim 6, wherein said polymer support is selected from the group consisting of polystyrene resin, PEG-g-PS resin, TentaGel™ resin, PEGA™ resin, CLEAR™ resin, epoxy resin, phenol resin, phenoxy resin, melamine resin, polyester resin, cellulose resin, agarose resin, chitosan resin and PMMA resin.

8. The method of Claim 6, wherein a solvent being used at said step i) is dichloromethane or dimethylformaldehyde.

9. The method of Claim 6, wherein a catalyst being used at said step i) is 4-dimethyl-aminopyridine.

10. The method of Claim 6, wherein a reaction temperature at said step i) is 0 - 50°C.

11. The method of Claim 6, wherein a reaction time at said step i) is 2 - 48 hours.

12. The method of Claim 6, wherein said seed of said step ii) is an amyloid seed.

13. The method of Claim 12, wherein said amyloid seed of said step ii) is a β-2-microglobulin amyloid seed.

14. The method of Claim 6, wherein a size of said seed is 1 nm to 10 µm.

15. A method for removing β-2-microglobulin, comprising: contacting said β-2-microglobulin amyloid seed-conjugated polymer support with a fluid comprising β-2-microglobulin.

16. The method of Claim 15, wherein a size of said β-2-microglobulin amyloid seed is 1 nm to 10 µm.

17. The method of Claim 15, wherein said β-2-microglobulin amyloid seed-conjugated polymer support is treated with one selected from the group consisting of bovine serum albumin, horse serum, human serum and skim milk.

18. The method of Claim 15, wherein said polymer support is selected from the group consisting of polystyrene resin, PEG-g-PS resin, TentaGel™ resin, PEGA™ resin, CLEAR™ resin, epoxy resin, phenol resin, phenoxy resin, melamine resin, polyester resin, cellulose resin, agarose resin, chitosan resin and PMMA resin.

19. The method of Claim 15, wherein said fluid further comprises phosphate buffer solution.

20. The method of Claim 15, wherein pH at the step of contacting is maintained between 6.0 and 8.0.

21. The method of Claim 15, wherein said fluid is mammalian blood.
